# DEMANDE DE BREVET EUROPEEN

(11) **EP 4 155 693 A1**
(43) Date de publication de la demande: **29.03.2023**
(21) Numéro de dépôt: 22197164.1
(22) Date de dépôt: 22.09.2022
(51) Int. Cl.: G01J 3/46, G01J 3/50, B01F 35/21, G01N 1/20, G01N 21/25, G01N 33/32, G05D 11/13, G01N 21/17, G01N 21/85, G01N 21/05, B05B 9/00

(54) **DISPOSITIF DE MESURE DE TEINTE POUR UN SYSTÈME DE DISTRIBUTION DE FLUIDE**

(30) Priorité: 23.09.2021 FR 2110045
(71) Demandeur: Exel Industries, 51200 Epernay (FR)
(72) Inventeur: BAIDAOUI, Ahmed, 91700 SAINTE GENEVIEVE DES BOIS (FR); KHALDI, Boussif, 95400 ARNOUVILLE (FR); DE TALHOUET, Philippe, 75116 PARIS (FR); FERRERE, Nicolas, 60460 PRECY SUR OISE (FR)
(74) Mandataire: Lavoix

(57) **Abrégé**

La présente invention vise un dispositif de mesure de teinte (10) configuré pour être connecté en série dans un circuit d'écoulement de fluide (120), comprenant au moins une source de lumière (1) configurée pour émettre une lumière polychromatique en direction du fluide dans une zone de mesure (140) ; un capteur de lumière (2) configuré pour recevoir un signal lumineux réfléchi sur le fluide ou transmis à travers le fluide, le signal lumineux réfléchi ou transmis correspondant à la réflexion ou la transmission optique, par le fluide, de la lumière polychromatique émise en direction du fluide par ladite au moins une source de lumière (1) ; une unité de calcul(3) configurée pour réaliser une analyse spectrale du signal lumineux reçu par le capteur de lumière (2) et pour déterminer une signature chromatique du fluide.

## Description

### DOMAINE TECHNIQUE

La présente invention concerne le domaine de la distribution de fluide, notamment de peinture par pulvérisation.

En particulier, la présente invention se rapporte à un dispositif de mesure de teinte adapté pour réaliser une identification de la teinte du fluide circulant dans un circuit d'écoulement de fluide destiné à être distribué, et en particulier pour superviser des opérations ou des phénomènes impliquant un changement de teinte dans un tel système de distribution de fluide.

### ETAT DE LA TECHNIQUE

Comme cela est connu, un système de distribution de fluide comprend, de manière générale, au moins un réservoir de fluide, un organe terminal de distribution, et un circuit d'écoulement de fluide acheminant le fluide depuis ledit au moins un réservoir de fluide jusqu'à l'organe terminal de distribution, par exemple un pistolet de pulvérisation.

Dans le contexte d'un système de distribution de peinture par pulvérisation, il est connu de réaliser en amont du pistolet de pulvérisation, un mélange de deux ou trois composants, par exemple d'une peinture et d'un catalyseur, le ratio optimal de la composition étant fourni par le fabricant de peinture. Il est intéressant de pouvoir surveiller le ratio de composition de la peinture avant sa pulvérisation, afin de pouvoir ajuster ce ratio de composition au besoin, par exemple au moyen d'un système de commande de valves.

En outre, lors d'une opération de changement de teinte, par exemple une montée en teinte, un changement de couleur, ou un rinçage du système de distribution de peinture par un solvant, il est connu de réaliser un contrôle visuel du fluide pulvérisé afin d'acter que l'opération de transition est achevée. Alternativement, il est connu d'acter que l'opération de transition est achevée sur la base d'une temporisation prédéfinie. De fait, la durée des opérations de changement de teinte n'est pas optimisée, ce qui induit du gaspillage et influe en conséquence sur les coûts d'achat des composants et sur les coûts de recyclage.

En réponse à ces inconvénients, la présente invention propose un dispositif de mesure de teinte configuré pour mesurer la teinte d'un fluide, en particulier pendant des opérations de changement de teinte. Ainsi, la présente invention permet avantageusement de limiter le gaspillage de fluide et de ses constituants.

### PRESENTATION DE L'INVENTION

Plus précisément, un aspect de l'invention a pour objet un dispositif de mesure de teinte configuré pour être connecté en série dans un circuit d'écoulement de fluide, le dispositif de mesure de teinte comprenant au moins une source de lumière, un capteur de lumière, et une unité de calcul. Ladite au moins une source de lumière est configurée pour émettre une lumière polychromatique en direction du fluide dans une zone de mesure. Le capteur de lumière est configuré pour recevoir un signal lumineux réfléchi sur le fluide ou transmis à travers le fluide, le signal lumineux réfléchi ou transmis correspondant à la réflexion optique ou, respectivement, la transmission optique, par le fluide, de la lumière polychromatique émise en direction du fluide par ladite au moins une source de lumière. L'unité de calcul est configurée pour réaliser une analyse spectrale du signal lumineux reçu par le capteur de lumière et pour déterminer une signature chromatique du fluide.

L'unité de calcul est en outre configurée pour déterminer le niveau de luminosité du signal lumineux reçu par le capteur de lumière.

Le dispositif de mesure de teinte selon l'invention permet de réaliser une analyse chromatique du fluide, et par conséquent, offre l'avantage considérable de permettre une identification rapide et fiable de la teinte du fluide, et une maîtrise améliorée des opérations de changement de teinte du fluide, notamment une montée en teinte, un changement de couleur de peinture, ou encore un rinçage dans le contexte d'un système de distribution de peinture. Ainsi, la présente invention permet une amélioration de l'efficacité de ces opérations de changement de teinte du fluide, et en conséquence de limiter substantiellement le gaspillage des composants fluides, réduisant ainsi les coûts d'achat ainsi que les coûts de recyclage. De plus, la connexion en série du dispositif de mesure de teinte au circuit d'écoulement de fluide permet avantageusement d'obtenir une mesure plus fiable de la teinte dans le circuit d'écoulement de fluide.

De manière préférée, le dispositif de mesure de teinte selon l'invention comprend au moins une fibre optique d'acquisition configurée pour acheminer le signal lumineux réfléchi sur le fluide ou transmis à travers le fluide à destination du capteur de lumière, le capteur de lumière étant déporté par rapport à la zone de mesure. Cette configuration présente l'avantage considérable d'éloigner les composants électroniques du capteur de lumière de la zone de mesure, pouvant être assujettie à une réglementation de type ATEX (pour « ATmosphère EXplosive »). Ainsi, il est possible d'utiliser un capteur de lumière standard, moins coûteux. De plus, le capteur de lumière est avantageusement éloigné d'éventuelles vibrations mécaniques du circuit d'écoulement de fluide, simplifiant encore la conception du capteur de lumière.

De manière préférée, le dispositif de mesure de teinte selon l'invention comprend une chambre, par exemple opaque, à travers laquelle est ménagée une veine d'écoulement de fluide configurée pour être raccordée de part et d'autre au circuit d'écoulement de fluide, la chambre, par exemple opaque, logeant une extrémité de chacune de ladite au moins une fibre optique d'acquisition. La chambre opaque permet, de manière avantageuse, de limiter fortement une éventuelle pollution lumineuse provenant du milieu extérieur lors des mesures de teintes.

Avantageusement, le dispositif de mesure de teinte selon l'invention comprend un corps d'extrémité encapsulant l'extrémité ou les extrémités de ladite au moins une fibre optique d'acquisition.

Le dispositif de mesure de teinte comprend en outre une fenêtre d'observation disposée de manière tangentielle à la veine d'écoulement de fluide.

Le dispositif de mesure de teinte comprend en outre un élément de maintien monté dans la chambre, par exemple opaque, de manière à maintenir en position la fenêtre d'observation et à garantir l'étanchéité de la veine d'écoulement de fluide.

Le corps d'extrémité est configuré pour s'insérer de manière amovible, ici dans la chambre, par exemple opaque, de manière à positionner le corps d'extrémité en regard de la fenêtre d'observation. Le corps d'extrémité offre l'avantage de simplifier les opérations de montage ou de démontage du corps d'extrémité. Par exemple, lors d'une opération de maintenance du dispositif de mesure de teinte, le corps d'extrémité peut être remplacé en temps « masqué » durant la pulvérisation du fluide, autrement dit sans perturber l'écoulement du fluide dans la veine.

Avantageusement, le dispositif de mesure de teinte selon l'invention comprend au moins une fibre optique d'éclairement reliée à une extrémité à ladite au moins une source de lumière et configurée pour acheminer la lumière polychromatique de ladite au moins une source de lumière en direction du fluide. De plus, le corps d'extrémité encapsule, de préférence, l'extrémité ou les extrémités de ladite au moins une fibre optique d'éclairement. Ainsi, la configuration du corps d'extrémité encapsulant, dans un même corps, lesdites au moins une fibre optique d'éclairement et au moins une fibre optique d'acquisition facilite la conception et la mise en œuvre du dispositif de mesure de teinte selon l'invention.

Avantageusement, le corps d'extrémité comprend un élément antireflet disposé de manière à isoler optiquement ladite au moins une fibre optique d'éclairement et ladite au moins une fibre optique d'acquisition entre elles, de manière à limiter la transmission directe de lumière de ladite au moins une fibre optique d'éclairement à ladite au moins une fibre optique d'acquisition. L'élément antireflet permet avantageusement de limiter la pollution lumineuse de ladite au moins une fibre optique d'acquisition par ladite au moins une fibre optique d'éclairement.

Avantageusement, le corps d'extrémité présentant un axe central, les extrémités desdites au moins une fibre optique d'éclairement et au moins une fibre optique d'acquisition sont respectivement réparties circulairement autour de l'axe central du corps d'extrémité à deux distances radiales différentes de l'axe central du corps d'extrémité. De plus, le corps d'extrémité comprend un évidement annulaire sur une face configurée pour venir en vis-à-vis de la zone de mesure, l'évidement annulaire étant aménagé dans une portion autour de l'axe central et ayant un rayon compris entre lesdites deux distances radiales différentes. Le corps d'extrémité comprend alors l'élément antireflet logé dans l'évidement annulaire de sorte à isoler optiquement ladite au moins une fibre optique d'éclairement et ladite au moins une fibre optique d'acquisition entre elles.

Selon un aspect de l'invention, l'invention concerne un système de distribution de fluide comprenant le circuit d'écoulement de fluide et le dispositif de mesure de teinte selon un autre aspect de l'invention décrit précédemment, le dispositif de mesure de teinte étant connecté en série dans le circuit d'écoulement de fluide.

Avantageusement, le système de distribution de fluide selon cet aspect de l'invention consiste en un système de distribution de peinture.

Selon un aspect de l'invention, l'invention se rapporte à un procédé de mesure de teinte d'un fluide circulant dans un circuit d'écoulement de fluide au moyen d'un dispositif de mesure de teinte comprenant au moins une source de lumière, un capteur de lumière, et une unité de calcul, le procédé comprenant les étapes suivantes :
- l'émission d'une lumière polychromatique en direction du fluide par ladite au moins une source de lumière ;
- la réception par le capteur de lumière du signal lumineux réfléchi sur le fluide ou transmis à travers le fluide ;
- la réalisation d'une analyse spectrale par l'unité de calcul du signal lumineux reçu par le capteur de lumière de sorte à déterminer une signature chromatique du fluide ;
- la comparaison de la signature chromatique du fluide à un abaque de signatures chromatiques cibles par l'unité de calcul de sorte à déterminer la teinte du fluide.

Avantageusement, la réalisation de l'analyse spectrale du signal lumineux comprend la décomposition du signal lumineux dans le système de couleurs RVB, la signature chromatique du fluide comprenant un code couleur RVB.

Avantageusement, le procédé de mesure de teinte selon cet aspect de l'invention, mis en œuvre lors d'une opération de changement de teinte du fluide dans le circuit d'écoulement de fluide, comprend la détermination que l'opération de changement de teinte est achevée lorsque la signature chromatique du fluide atteint une signature chromatique cible pendant une durée prédéfinie, la signature chromatique cible consistant en un sous-ensemble de l'abaque de signatures chromatiques cibles.

Selon un mode de réalisation de l'invention, le procédé comprend, en outre, la détermination du niveau de luminosité ou *clear* à partir du signal lumineux reçu par le capteur de lumière, plus particulièrement par l'unité de calcul.

Le procédé de mesure de teinte selon ce mode de réalisation de l'invention, mis en œuvre lors d'une opération de rinçage et/ou de montée de teinte dans le circuit d'écoulement de fluide, comprend la détermination que l'opération est achevée lorsque la signature chromatique du fluide atteint une signature chromatique cible pendant une durée prédéfinie et/ou que le niveau de luminosité atteint une valeur souhaitée.

Selon un aspect de l'invention, l'invention se rapporte à un procédé de distribution de peinture comprenant le procédé de mesure de teinte décrit précédemment, l'opération de changement de teinte du fluide dans le circuit d'écoulement de fluide comprenant une opération de montée en teinte, de changement de couleur, ou de rinçage.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
- la figure 1 est une représentation schématique d'un système de distribution de fluide comprenant un dispositif de mesure de teinte selon un premier mode de réalisation de l'invention ;
- la figure 2 est une représentation schématique d'un dispositif de mesure de teinte selon un deuxième mode de réalisation de l'invention ;
- la figure 3 est une représentation schématique d'un dispositif de mesure de teinte selon un troisième mode de réalisation de l'invention ;
- la figure 4 est une représentation schématique d'un dispositif de mesure de teinte selon un quatrième mode de réalisation de l'invention ;
- la figure 5A est une représentation schématique d'une vue en coupe partielle d'un exemple de dispositif de mesure de teinte selon le premier mode de réalisation de l'invention ;
- la figure 5B est une représentation schématique d'une vue partielle d'un autre exemple de dispositif de mesure de teinte selon un autre mode de réalisation de l'invention ;
- la figure 6 est une représentation schématique d'une vue d'un corps d'extrémité d'un exemple de dispositif de mesure de teinte selon l'invention ;
- la figure 7 est une représentation schématique d'un procédé de mesure de teinte selon un aspect de l'invention ;
- la figure 8 est une représentation schématique de l'évolution temporelle des signatures chromatiques lors d'opérations de changement de teinte.

Il faut noter que les figures exposent l'invention de manière détaillée pour permettre de mettre en œuvre l'invention, lesdites figures pouvant bien entendu servir à mieux définir l'invention le cas échéant.

### DESCRIPTION DETAILLEE DE L'INVENTION

L'invention concerne un dispositif de mesure de teinte pour un système de distribution de fluide, le fluide pouvant présenter une large gamme de viscosité, d'une viscosité faible à une viscosité élevée. L'invention est décrite ci-après dans le contexte d'un système de distribution de peinture, en particulier par pulvérisation. Cependant, cela ne constitue pas une limitation de l'invention à cette application particulière, l'invention pouvant être mise en œuvre dans tout système de distribution de fluide adapté pour cheminer un fluide, dans lequel il est avantageux d'identifier la teinte du fluide, notamment pour superviser des opérations ou des phénomènes impliquant un changement de teinte du fluide.

De manière générale, le système de distribution de peinture comprend au moins un réservoir de composants de la peinture, un organe terminal de distribution, également désigné tête de distribution, et un circuit d'écoulement de fluide ayant des canalisations et mettant en liaison fluidique ledit au moins un réservoir de fluide à l'organe terminal de distribution via les tuyaux. L'organe terminal de distribution peut par exemple comprendre un pistolet de pulvérisation.

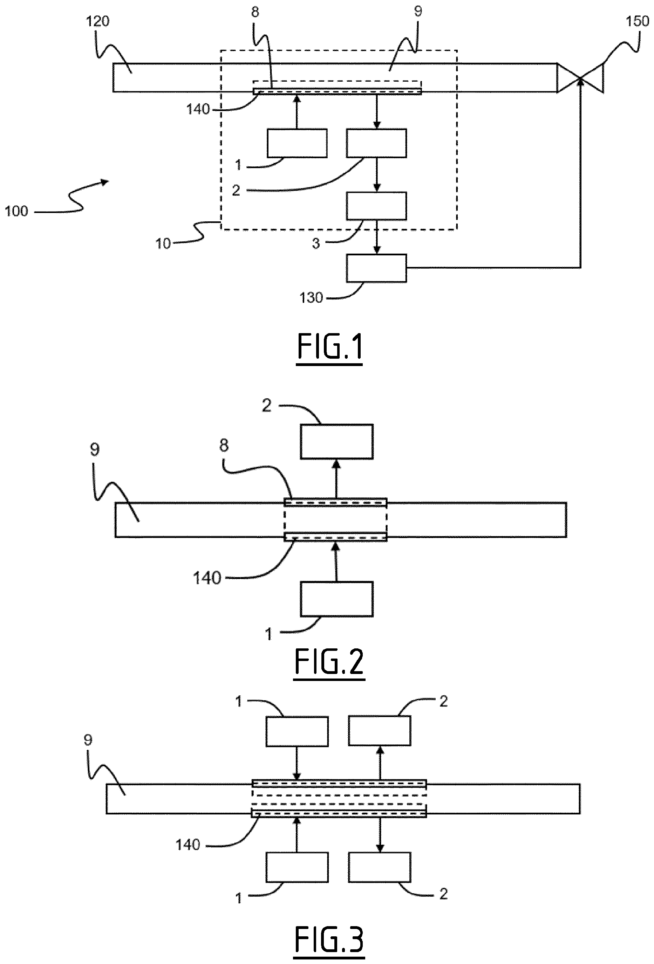

En référence à la figure 1, selon un aspect de l'invention, l'invention concerne le dispositif de mesure de teinte 10 configuré pour être connecté en série dans un circuit d'écoulement de fluide 120. Le dispositif de mesure de teinte 10 selon cet aspect de l'invention comprend au moins une source de lumière 1 configurée pour émettre une lumière polychromatique en direction du fluide dans une zone de mesure 140, un capteur de lumière 2 configuré pour recevoir un signal lumineux réfléchi sur le fluide ou transmis à travers le fluide, et une unité de calcul 3 configurée pour réaliser une analyse spectrale du signal lumineux reçu par le capteur de lumière 2 afin de déterminer une signature chromatique du fluide. Le signal lumineux réfléchi ou transmis correspond à la réflexion optique ou, respectivement, la transmission optique, par le fluide, de la lumière polychromatique émise en direction du fluide par ladite au moins une source de lumière
1. Dans un mode de réalisation préférentiel, la lumière polychromatique est de la lumière blanche. En variante, la source de lumière 1 peut comporter une pluralité de sources monochromatiques de longueurs d'ondes respectives différentes, par exemple une source laser rouge, une source laser verte, et une source laser bleue.

Dans un mode de réalisation, l'unité de calcul est en outre configurée pour déterminer le niveau de luminosité du signal lumineux reçu par le capteur de lumière 2.

L'analyse, notamment comprenant l'analyse chromatique, du fluide tel que le permet l'invention offre l'avantage considérable de permettre une identification rapide et fiable de la teinte du fluide, et par conséquent une maîtrise améliorée des opérations de changement de teinte du fluide, notamment une montée en teinte, un changement de couleur de peinture, ou encore un rinçage du système de distribution de peinture par un solvant, par exemple de l'eau. La présente invention offre l'avantage considérable de permettre une amélioration de l'efficacité de ces opérations de changement de teinte du fluide, et en conséquence de limiter substantiellement le gaspillage des composants de peinture, et de solvant, réduisant ainsi les coûts d'achat ainsi que les coûts de recyclage.

Notamment, la connexion en série du dispositif de mesure de teinte au circuit d'écoulement de fluide permet avantageusement d'obtenir une mesure plus fiable de la teinte dans le circuit d'écoulement de fluide.

La lumière polychromatique consiste de préférence en une lumière blanche, et ladite au moins une source de lumière consiste, de façon préférée, en une ou plusieurs diodes électroluminescentes, également désignées LED. De plus, ladite au moins une source de lumière peut être configurée pour émettre une lumière puisée.

Le capteur de lumière consiste de préférence en un capteur de couleurs, en particulier un capteur de couleurs RVB (pour « Rouge Vert Bleu »).

En variante, le capteur de lumière est apte à capter la couleur, par exemple les couleurs RVB, mais également le niveau de luminosité.

Il est possible de positionner le capteur de lumière à proximité de la zone de mesure. Toutefois, la qualité du fluide distribué ou son environnement peuvent souvent induire des contraintes de respect d'une réglementation de type ATEX (pour « ATmosphère EXplosive »), comme dans le contexte du système de distribution de peinture. Or l'utilisation d'un capteur de lumière compatible avec cette contrainte peut se révéler coûteux et son intégration au dispositif de mesure de teinte peut s'avérer complexe, voire impossible.

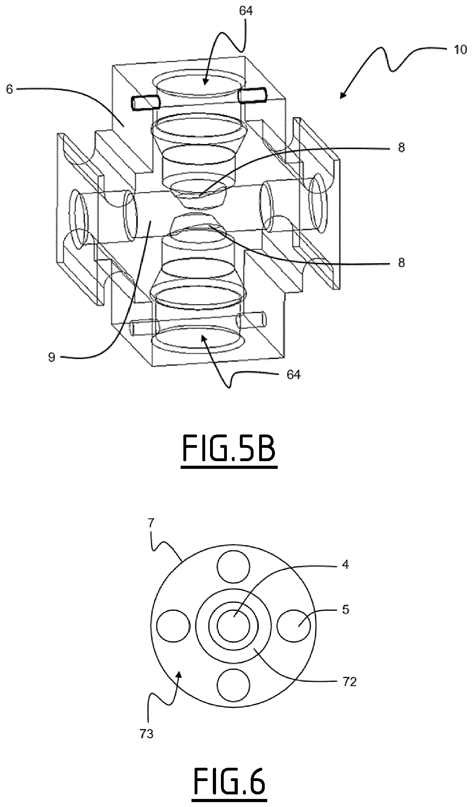

Pour répondre à ces contraintes, le dispositif de mesure de teinte selon l'invention peut comprendre, de manière préférée, au moins une fibre optique d'acquisition 4, par exemple une seule fibre optique d'acquisition 4 comme représenté sur la figure 6, configurée pour acheminer le signal lumineux réfléchi sur le fluide ou transmis à travers le fluide à destination du capteur de lumière 2. De plus, le capteur de lumière 2 est alors configuré pour être déporté par rapport à la zone de mesure. Cette configuration présente l'avantage considérable d'éloigner les composants électroniques du capteur de lumière 2 de la zone ATEX, et ainsi de pouvoir utiliser un capteur de lumière standard. De plus, le capteur de lumière est avantageusement éloigné d'éventuelles vibrations mécaniques du circuit d'écoulement de fluide, ce qui simplifie encore la conception du capteur de lumière.

En outre, la mise en place d'au moins une fibre optique d'acquisition et du capteur de lumière déporté par rapport à la zone de mesure permet avantageusement de mutualiser un capteur de lumière entre une pluralité de zones de mesures distinctes. Il faut toutefois, le cas échéant, prévoir un module de séquencement, dans le dispositif selon l'invention, configuré pour que les émissions de lumière, par ladite au moins une source de lumière, soient synchronisés, afin d'éviter que les signaux lumineux réceptionnés par le capteur de lumière ne se croisent, faussant conséquemment la mesure.

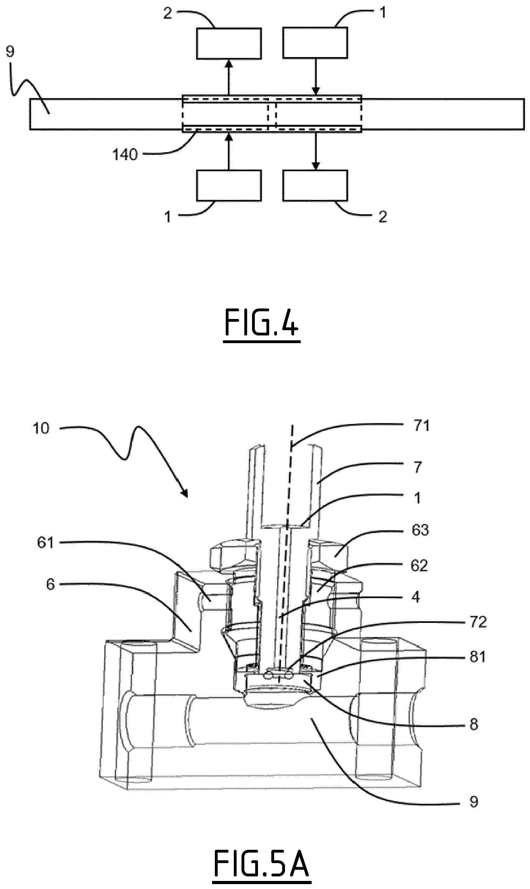

De plus, en référence à la figure 5A, le dispositif de mesure de teinte comprend de préférence une chambre, par exemple opaque, 6 à travers laquelle est ménagée une veine d'écoulement de fluide 9, agencée pour être connectée de part et d'autre au circuit d'écoulement de fluide 120. En particulier, la chambre, par exemple opaque, 6 loge une extrémité de chacune de ladite au moins une fibre optique d'acquisition 4. La chambre opaque 6 permet, de manière avantageuse, de limiter fortement une éventuelle pollution lumineuse provenant du milieu extérieur lors des mesures de teintes. De plus, la chambre, par exemple opaque, 6 comprend notamment des moyens de maintien de l'extrémité ou des extrémités correspondantes, par exemple une gaine.

En référence à la figure 6, le dispositif de mesure de teinte 10 peut également comprendre au moins une fibre optique d'éclairement 5 reliée à une extrémité à ladite au moins une source de lumière et configurée pour acheminer la lumière polychromatique de ladite au moins une source de lumière en direction du fluide. Le nombre de fibres optiques d'éclairement 5 et le nombre de sources de lumière sont notamment adaptés pour fournir une intensité lumineuse suffisante afin que le signal lumineux réfléchi ou transmis présente en retour une intensité lumineuse suffisante pour effectuer la mesure de teinte. Le nombre de fibres optiques d'éclairement 5 peut notamment correspondre au nombre de sources de lumière, par exemple quatre.

Les figures 1 à 4 représentent différents agencements possibles de ladite au moins une source de lumière 1 et dudit capteur de lumière 2.

En particulier, selon un premier mode de réalisation de l'invention représenté sur la figure 1, l'analyse du fluide est réalisée sur la base d'un signal lumineux réfléchi sur le fluide. Dans ce premier mode de réalisation de l'invention, l'émission de lumière par ladite au moins une source de lumière et la réception du signal lumineux réfléchi sur le fluide sont réalisées d'un même côté de la veine d'écoulement de fluide 9. Une telle configuration induit une mesure de la teinte substantiellement en surface du fluide. Ce premier mode de réalisation de l'invention offre l'avantage de présenter une compacité élevée du dispositif de mesure de teinte, et de faciliter l'intégration du dispositif de mesure de teinte au système de distribution de peinture, notamment via l'étendue réduite de la zone de mesure à une portion limitée de la veine d'écoulement de fluide 9.

Selon un deuxième mode de réalisation de l'invention, représenté sur la figure 2, l'analyse du fluide est réalisée sur la base du signal lumineux transmis à travers le fluide. Dans ce deuxième mode de réalisation de l'invention, l'émission de lumière par ladite au moins une source de lumière et la réception du signal lumineux transmis à travers le fluide sont réalisées respectivement de part et d'autre de la veine d'écoulement de fluide 9. Cette configuration permet de réaliser une mesure de teinte à travers une section du fluide, et améliore en conséquence la précision de la mesure de teinte, au détriment toutefois de la compacité du dispositif de mesure de teinte, et de l'intégration du dispositif de mesure de teinte au système de distribution de peinture.

La présente invention permet également de réaliser des combinaisons du premier et du deuxième modes de réalisation de l'invention décrits précédemment. A titre d'exemple, les figures 3 et 4 représentent, de manière schématique, respectivement un troisième et un quatrième modes de réalisation de l'invention dans lesquels l'émission de lumière par ladite au moins une source de lumière et la réception du signal lumineux réfléchi ou transmis sont chacune réalisées des deux côtés de la veine d'écoulement de fluide 9. Selon le troisième mode de réalisation de l'invention, le signal lumineux réfléchi à la surface du fluide est mesuré de part et d'autre de la veine d'écoulement de fluide 9. Selon le quatrième mode de réalisation de l'invention, le signal lumineux transmis à travers le fluide est également mesuré de part et d'autre de la veine d'écoulement de fluide 9. Dans ces deux cas de figures, il peut être avantageux d'émettre une lumière pulsée et synchronisée afin d'éviter que les signaux lumineux réfléchis ou transmis ne se croisent.

Les modes de réalisation mettant en œuvre de la transmission du signal lumineux à travers le fluide permettent notamment de limiter l'impact des gradients de teinte dans l'épaisseur de la veine d'écoulement de fluide sur les mesures de teinte, en particulier lorsque l'épaisseur de la veine d'écoulement de fluide ou la viscosité du fluide sont élevées.

L'invention va maintenant être détaillée dans le contexte de la configuration non-limitative du premier mode de réalisation de l'invention, à savoir que l'émission de lumière par ladite au moins une source de lumière et la réception du signal lumineux réfléchi sur le fluide sont réalisées d'un même côté de la veine d'écoulement de fluide. On peut noter que les éléments décrits ci-après sont transposables au contexte d'une configuration mettant en œuvre la mesure de teinte par transmission optique du signal lumineux à travers le fluide.

De manière préférée, comme représenté sur la figure 5A, le dispositif de mesure de teinte comprend un corps d'extrémité 7 encapsulant les extrémités desdites au moins une fibre optique d'éclairement 5 et au moins une fibre optique d'acquisition 4.

Alternativement, le corps d'extrémité 7 peut uniquement encapsuler l'extrémité ou les extrémités de ladite au moins une fibre optique d'acquisition 4. Cependant, la configuration du corps d'extrémité encapsulant, dans un même corps, lesdites au moins une fibre optique d'éclairement 5 et d'acquisition 4 facilite la conception et la mise en œuvre du dispositif de mesure de teinte selon l'invention.

La ou lesdites extrémités sont encapsulées dans le corps d'extrémité de sorte qu'elles débouchent au niveau d'une face 73 du corps d'extrémité configurée pour venir en vis-à-vis de la zone de mesure 140.

Le dispositif de mesure de teinte comprend, en outre, une fenêtre d'observation 8 disposée de manière tangentielle à la veine d'écoulement de fluide 9. La fenêtre d'observation 8 comprend, par exemple, une lentille.

La fenêtre d'observation 8 est apte à résister à une pression de sécurité supérieure ou égale à 1000 bar.

Le dispositif de mesure de teinte comprend, en outre, un élément de maintien 81 monté dans la chambre, par exemple opaque, 6 de manière à maintenir en position la fenêtre d'observation 8 et à garantir l'étanchéité de la veine d'écoulement de fluide 9.

La fenêtre d'observation 8 peut notamment présenter la forme d'un hublot, comme observé sur la figure 5A illustrant un exemple de réalisation du dispositif de mesure de teinte selon l'invention.

L'élément de maintien 81 peut, à titre d'exemple, comprendre un joint d'étanchéité encerclant la fenêtre d'observation 8.

Le corps d'extrémité 7 est configuré pour s'insérer, au moins partiellement, notamment de manière amovible, par exemple dans la chambre, par exemple opaque 6, de manière à positionner le corps d'extrémité 7 en regard de la fenêtre d'observation 8. Le corps d'extrémité 7 présente avantageusement une forme de crayon, autrement dit une forme sensiblement tubulaire de sorte à simplifier les opérations de montage ou de démontage du corps d'extrémité, par exemple pour la maintenance du dispositif de mesure de teinte. Ainsi, le corps d'extrémité 7 permet avantageusement d'effectuer une maintenance du dispositif de mesure de teinte en temps « masqué » durant la pulvérisation du fluide, autrement dit sans perturber l'écoulement du fluide dans la veine.

En référence à la figure 5A, le corps d'extrémité 7 peut avantageusement se fixer à la chambre, par exemple opaque, 6 en se vissant à un boulon 63 par exemple. De manière préférée, le corps d'extrémité 7 est configuré pour se visser uniquement sur un quart de tour, afin d'éviter d'emmêler les fibres optiques et de faciliter le montage du dispositif de mesure de teinte selon l'invention. Pour améliorer le maintien du corps d'extrémité 7, la chambre, par exemple opaque, 6 peut comprendre un élément de blocage mécanique 61, par exemple une goupille, venant empêcher la rotation du corps d'extrémité 7.

De plus, en référence à la figure 5A, la chambre, par exemple opaque, 6 comprend en particulier un presse-étoupe 62 afin d'assurer le maintien des fibres optiques.

Alternativement au boulon 63, le corps d'extrémité 7 est pourvu d'un écrou fou ou tournant, plus particulièrement sur une portion distale du corps d'extrémité 7, la portion distale étant agencée à l'opposé des extrémités des fibres ou de la face 73.

L'écrou fou ou tournant est solidaire du corps d'extrémité 7, mais ne transmet pas audit corps d'extrémité 7 tout mouvement de rotation de vissage ou serrage appliqué à l'écrou dans un fonctionnement normal de l'écrou fou ou tournant.

L'écrou fou ou tournant est prévu pour être vissé sur la chambre 6.

Un tel écrou fou ou tournant permet d'éviter toute torsion des fibres, dans la mesure où le mouvement pour visser l'écrou fou ou tournant à la chambre 6 n'est pas transmis aux fibres.

Par ailleurs, le corps d'extrémité 7 comprend, de préférence, un élément antireflet disposé de manière à isoler optiquement ladite au moins une fibre optique d'éclairement 5 et ladite au moins une fibre optique d'acquisition 4 entre elles, de manière à limiter la transmission directe de lumière de ladite au moins une fibre optique d'éclairement 5 à ladite au moins une fibre optique d'acquisition 4.

L'élément antireflet permet avantageusement de limiter la pollution lumineuse des fibres optiques d'acquisition 4 par les fibres optiques d'éclairement 5. Plus particulièrement, un tel élément antireflet limite la pollution lumineuse de la mesure réalisée à partir de l'au moins une fibre optique d'acquisition par de la lumière qui serait réfléchie directement de l'au moins une fibre optique d'éclairement vers l'au moins une fibre optique d'acquisition, sans être réfléchie sur le fluide.

L'élément antireflet est, par exemple, un joint torique, plus particulièrement en caoutchouc ou élastomère.

De manière préférée, en référence aux figures 5A et 6, le corps d'extrémité 7 présente un axe central 71. Alors, les extrémités desdites au moins une fibre optique d'éclairement 5 et au moins une fibre optique d'acquisition 4 sont respectivement réparties circulairement autour de l'axe central 71 du corps d'extrémité 7 à deux distances radiales différentes de l'axe central 71 du corps d'extrémité 7.

L'au moins une fibre optique d'acquisition 4 est, par exemple, une unique fibre d'acquisition dont l'extrémité est agencée sur l'axe central 71. L'au moins une fibre optique d'éclairement 5 comprend ici une pluralité de fibres d'éclairement réparties autour de l'axe central 71 et agencées sur un cercle centré sur l'axe central 71.

L'élément antireflet est agencé entre les extrémités de l'au moins une fibre optique d'éclairement 5 d'une part et les extrémités de l'au moins une fibre d'acquisition 4 d'autre part. Plus particulièrement, l'élément antireflet est agencé autour de l'axe central 71 entre les deux distances radiales.

Dans un mode de réalisation représenté sur la figure 6, le corps d'extrémité 7 comprend un évidement annulaire 72 sur une face 73 configurée pour venir en vis-à-vis de la zone de mesure 140, l'évidement annulaire 72 étant aménagé dans une portion autour de l'axe central 71 et ayant un rayon compris entre lesdites deux distances radiales. Le corps d'extrémité 7 comprend alors l'élément antireflet, par exemple un joint, logé dans l'évidement annulaire 72 de sorte à isoler optiquement ladite au moins une fibre optique d'éclairement 5 et ladite au moins une fibre optique d'acquisition 4 entre elles.

Alternativement, le corps d'extrémité 7 comprend un évidement présentant la forme d'un disque sur la face 73 configurée pour venir en vis-à-vis de la zone de mesure 140. L'évidement s'étend ainsi en retrait du reste de la face 73.

L'évidement est centré sur l'axe central 71 et présente un rayon compris entre lesdites deux distances radiales.

La ou les extrémités de l'au moins une fibre optique d'acquisition 4 ou d'éclairement 5 agencée à la distance radiale la plus faible est ou sont en retrait par rapport à la ou les extrémités de l'autre de l'au moins une fibre optique d'acquisition 4 ou d'éclairement 5. Dans l'exemple décrit précédemment, l'extrémité de la fibre optique d'acquisition 4 selon l'axe central 71 débouche au niveau de l'évidement et est est agencée en retrait des extrémités des fibres optiques d'éclairement 5.

L'élément antireflet, ici le joint torique, est logé dans l'évidement. Plus particulièrement, l'évidement présente un rayon sensiblement égal au rayon externe du joint torique. Le joint torique est inséré dans l'évidement et est maintenu dans l'évidement par contact avec les rebords de l'évidement.

En outre, l'élément antireflet présente une hauteur, mesurée selon la direction de l'axe central 71 lorsque l'élément antireflet est inséré dans l'évidement, annulaire 72 ou en forme de disque, de sorte que l'élément antireflet dépasse de la face 73 en l'absence de contraintes supplémentaires. Plus particulièrement, l'élément antireflet a une hauteur strictement supérieure à la hauteur de l'évidement.

Lorsque le corps d'extrémité est fixé à la chambre, la face 73 s'étend en regard de la fenêtre d'observation 8, plus particulièrement de la lentille de la fenêtre d'observation 8.

Plus particulièrement, lors de fixation du corps d'extrémité à la chambre, notamment par boulon 63 ou par écrou fou ou tournant, une force est appliquée sur le corps d'extrémité 7 vers la fenêtre d'observation 8. Ainsi, lorsque la fixation est serrée, notamment par serrage du boulon 63 ou de l'écrou fou ou tournant, le joint torique se presse entre l'évidement et la face 73. Ainsi, aucune pollution lumineuse est susceptible de passer à ce niveau. La face 73 entre, par exemple, en contact avec la fenêtre d'observation après serrage.

Comme exprimé précédemment, le dispositif de mesure de teinte selon l'invention peut également être configuré pour réaliser une mesure de teinte par transmission du signal lumineux à travers le fluide. Le cas échéant, comme représenté sur la figure 5B, le dispositif de mesure de teinte 10 comprend avantageusement deux corps d'extrémité encapsulant chacun des extrémités de fibres optiques d'éclairement et/ou d'acquisition.

Le dispositif de mesure de teinte comprend en outre deux fenêtres d'observation 8 disposées de manière tangentielle à la veine d'écoulement de fluide 9 et en vis-à-vis l'une de l'autre.

Les deux corps d'extrémité sont agencés en regard l'un de l'autre, ici selon l'axe central de chaque corps d'extrémité, qui sont ici confondus, chaque corps d'extrémité s'étendant en regard d'une fenêtre d'observation respective.

Le dispositif de mesure de teinte 10 comprend, par exemple, deux corps d'extrémité tels que décrits précédemment. Dans un mode de réalisation alternatif, le corps d'extrémité 7 est, dans cette configuration, dépourvu d'élément antireflet.

Lors de la mesure, l'au moins une fibre optique d'éclairement d'un premier des corps d'extrémité est activée et la mesure est réalisée sur l'au moins une fibre d'acquisition du deuxième des corps d'extrémité. L'au moins une fibre optique d'éclairement du deuxième des corps d'extrémité est, par exemple, désactivée, c'est-à-dire que la source lumineuse correspondante est éteinte.

Les corps d'extrémité sont configurés pour s'insérer, au moins partiellement, notamment de manière amovible, dans la chambre, par exemple opaque, 6, de façon à permettre le positionnement de chacun des deux corps d'extrémité en regard de la fenêtre d'observation correspondante. Les deux corps d'extrémité sont notamment configurés pour s'insérer dans deux évidements 64 respectifs de la chambre, ici opaque 6.

Dans un mode de réalisation, l'opacité de la chambre 6 empêche notamment la pollution de la mesure par la lumière provenant de l'environnement extérieur au dispositif.

De plus, dans un tel dispositif de mesure de teinte configuré pour réaliser une mesure de teinte par transmission du signal lumineux à travers le fluide, les corps d'extrémité peuvent avantageusement présenter des caractéristiques semblables à celles décrites précédemment dans le contexte du premier mode de réalisation de l'invention. De la même manière, le dispositif de mesure de teinte peut comprendre deux éléments de maintien montés dans la chambre de manière à maintenir en position respectivement chacune des deux fenêtres d'observation 8 et à garantir l'étanchéité de la veine d'écoulement de fluide 9. La fixation des deux corps d'extrémités à la chambre peut être également réalisée comme décrit précédemment, en particulier au moyen d'un boulon ou d'un écrou fou, d'un élément de blocage mécanique, et d'un presse étoupe.

De plus, en référence à la figure 1, le dispositif de mesure de teinte peut être configuré pour transmettre les résultats de l'analyse du fluide à une unité de commande 130 configurée pour commander une valve ou un ensemble de valves 150 du système de distribution de peinture en fonction des résultats de l'analyse spectrale. Ainsi, il est possible, par exemple, d'optimiser les opérations de changement de teinte, ou d'ajuster la composition de la peinture.

Selon un autre aspect de l'invention, en référence à la figure 1, l'invention concerne un système de distribution de fluide 100 comprenant un circuit d'écoulement de fluide 120 et un dispositif de mesure de teinte 10 selon l'invention connecté en série du circuit d'écoulement de fluide 120. Par exemple, comme décrit précédemment, le système de distribution de fluide 100 peut consister en un système de distribution de peinture.

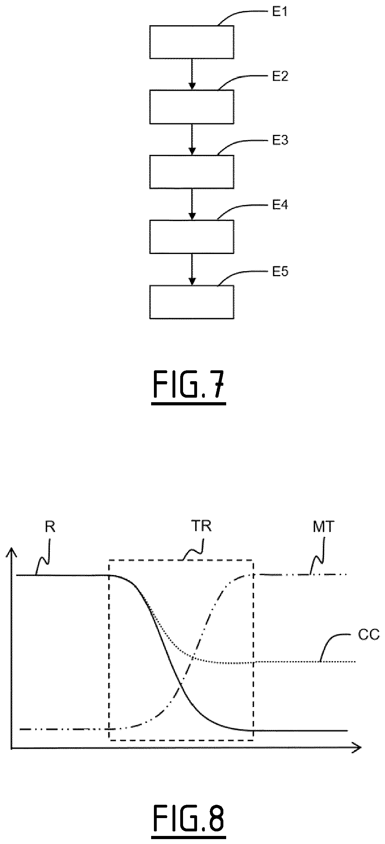

Selon un autre aspect de l'invention, comme représenté sur la figure 7, l'invention concerne un procédé de mesure de teinte d'un fluide circulant dans un circuit d'écoulement de fluide au moyen du dispositif de mesure de teinte tel que décrit précédemment.

Le procédé de mesure de teinte selon cet autre aspect de l'invention comprend les étapes suivantes :
- l'émission E1 d'une lumière polychromatique en direction du fluide par ladite au moins une source de lumière ;
- la réception E2 par le capteur de lumière du signal lumineux réfléchi sur le fluide ou transmis à travers le fluide ;
- la réalisation E3 d'une analyse spectrale par l'unité de calcul du signal lumineux reçu par le capteur de lumière de sorte à déterminer une signature chromatique du fluide ;
- la comparaison E4 de la signature chromatique du fluide à un abaque de signatures chromatiques cibles par l'unité de calcul 3 de sorte à déterminer la teinte du fluide.

Dans un mode de réalisation, l'analyse du fluide comprend en outre la détermination du niveau de luminosité du signal lumineux reçu par le capteur de lumière, ici par l'unité de calcul.

Les étapes précédentes peuvent avantageusement être répétées de manière régulière à une fréquence prédéterminée, de sorte à obtenir une évolution temporelle de la signature chromatique du fluide, et éventuellement du niveau de luminosité.

Avantageusement, la réalisation de l'analyse spectrale E3 du signal lumineux comprend la décomposition du signal lumineux dans le système de couleurs RVB, la signature chromatique du fluide comprenant un code couleur RVB, correspondant à un instant donné. Ainsi, on peut obtenir une évolution temporelle du code couleur RVB du fluide.

Dans le contexte de l'utilisation du système de couleurs RVB, ledit ensemble de signatures chromatiques cibles comprend le tableau des codes de couleurs RVB.

De plus, il est avantageux de mettre en œuvre le procédé de mesure de teinte lors d'une opération de changement de teinte du fluide dans le circuit d'écoulement de fluide. Alors, en référence à la figure 7, le procédé de mesure de teinte peut comprendre la détermination E5 que l'opération de changement de teinte est achevée lorsque la signature chromatique du fluide atteint une signature chromatique cible pendant une durée prédéfinie, la signature chromatique cible consistant en un sous-ensemble de l'abaque des signatures chromatiques cibles.

Dans un mode de réalisation, le procédé de mesure de teinte, mis en œuvre lors d'une opération de rinçage et/ou de montée de teinte dans le circuit d'écoulement de fluide, comprend la détermination que l'opération est achevée lorsque la signature chromatique du fluide atteint une signature chromatique cible pendant une durée prédéfinie, tel que décrit précédemment, et/ou que le niveau de luminosité atteint une valeur souhaitée prédéfinie par l'utilisateur (seuil).

Selon un autre aspect de l'invention, l'invention concerne un procédé de distribution de peinture comprenant le procédé de mesure de teinte décrit précédemment.

Comme décrit précédemment, les opérations de changement de teinte du fluide dans le circuit d'écoulement de fluide comprennent, entre autres, les opérations de montée en teinte, de changement de couleur, ou encore de rinçage.

La détection par transmission est, par exemple, mise en œuvre pour un fluide peu opaque, en particulier lors du rinçage.

La détection par réflexion est, par exemple, mise en œuvre pour un fluide opaque, par exemple lors de la montée en teinte ou un mélange de produits.

La figure 8 propose une illustration schématique d'évolutions temporelles de la signature chromatique du fluide pour différentes opérations de changement de teinte menées dans la zone de transition TR. Par exemple, la montée en teinte MT correspond à la mise en circulation d'une première couleur à la place d'un solvant initial dans le circuit d'écoulement de fluide. Dans un deuxième exemple, le changement de couleur CC correspond à une transition d'une première à une deuxième couleur. Dans un troisième exemple, le rinçage R correspond à la mise en circulation d'un solvant afin de nettoyer le circuit d'écoulement de fluide de la peinture. La présente invention présente l'avantage substantiel de pouvoir optimiser le rinçage, qui est une opération critique, car un mauvais rinçage peut détériorer le système de distribution de peinture, et pouvant durer longuement. De plus, concernant le rinçage, le procédé de mesure de teinte permet avantageusement de surveiller la couleur du solvant et d'estimer si une partie du solvant peut être éventuellement réutilisée dans un souci d'économie de matière.

Par ailleurs, le procédé de mesure de teinte peut également permettre d'identifier la présence de bulles d'air dans le fluide, afin par exemple de surveiller le démarrage et l'arrêt de la pompe configurée pour assurer l'écoulement de fluide. La présence de bulles d'air dans le fluide se manifeste par des instabilités dans les données brutes de l'évolution temporelle du code couleur RVB du fluide et de la luminosité.

En résumé, la présente invention offre l'avantage considérable de permettre d'optimiser de manière automatique les opérations de changement de teinte. En conséquence, la durée des opérations de changement de teinte est réduite et le gaspillage des composants du fluide est limitée.

Par ailleurs, l'invention peut également permettre de vérifier la composition du mélange du fluide, pour des mélanges ayant un nombre limité de composants comme pour la peinture, par exemple un mélange de deux ou trois composants. Dans le contexte d'une peinture, l'invention rend possible de surveiller le mélange couleur et catalyseur.

De plus, le dispositif de mesure de teinte selon l'invention présente une autonomie améliorée. Par exemple, il est connu de réaliser un système de distribution de peinture conventionnel où les opérations de changement de teinte sont menées sur une durée prédéterminée. La détermination de cette durée est complexe, car elle dépend de plusieurs paramètres, dont les fluides en jeu, ou encore le volume des canalisations du circuit d'écoulement de fluide. Ainsi, en comparaison d'un tel système de distribution de peinture conventionnel, l'utilisateur n'a plus à reprogrammer les opérations de changement de teinte en fonction des spécificités des fluides en jeu.

L'invention peut également permettre de réaliser un tri automatique selon la teinte de fluides, par exemple pour réaliser un tri des déchets intelligent.

## Revendications

1. Dispositif de mesure de teinte (10) configuré pour être connecté en série dans un circuit d'écoulement de fluide (120), le dispositif de mesure de teinte (10) comprenant :
- au moins une source de lumière (1) configurée pour émettre une lumière polychromatique en direction du fluide dans une zone de mesure (140) ;
- un capteur de lumière (2) configuré pour recevoir un signal lumineux réfléchi sur le fluide ou transmis à travers le fluide, le signal lumineux réfléchi ou transmis correspondant à la réflexion optique ou, respectivement, la transmission optique, par le fluide, de la lumière polychromatique émise en direction du fluide par ladite au moins une source de lumière (1) ;
- une unité de calcul (3) configurée pour réaliser une analyse spectrale du signal lumineux reçu par le capteur de lumière (2) et pour déterminer une signature chromatique du fluide.

2. Dispositif de mesure de teinte (10) selon la revendication précédente, **caractérisé en ce qu'**il comprend au moins une fibre optique d'acquisition (4) configurée pour acheminer le signal lumineux réfléchi sur le fluide ou transmis à travers le fluide à destination du capteur de lumière (2), le capteur de lumière (2) étant déporté par rapport à la zone de mesure (140).

3. Dispositif de mesure de teinte (10) selon la revendication 2, **caractérisé en ce qu'**il comprend une chambre opaque (6) à travers laquelle est ménagée une veine d'écoulement de fluide (9) configurée pour être raccordée de part et d'autre au circuit d'écoulement de fluide (120), la chambre opaque (6) logeant une extrémité de chacune de ladite au moins une fibre optique d'acquisition (4).

4. Dispositif de mesure de teinte (10) selon la revendication 2 ou 3, **caractérisé en ce qu'**il comprend un corps d'extrémité (7) encapsulant l'extrémité ou les extrémités de ladite au moins une fibre optique d'acquisition (4).

5. Dispositif de mesure de teinte (10) selon les revendications 2 et 4, comprenant une fenêtre d'observation (8) disposée de manière tangentielle à la veine d'écoulement de fluide (9), et un élément de maintien (81) monté dans la chambre opaque (6) de manière à maintenir en position la fenêtre d'observation (8) et à garantir l'étanchéité de la veine d'écoulement de fluide (9), le corps d'extrémité (7) étant configuré pour s'insérer de manière amovible dans la chambre opaque (6), de préférence dans une presse étoupe (62) de la chambre opaque (6), de manière à positionner le corps d'extrémité (7) en regard de la fenêtre d'observation (8).

6. Dispositif de mesure de teinte (10) selon la revendication 4 ou 5, **caractérisé en ce qu'**il comprend au moins une fibre optique d'éclairement (5) reliée à une extrémité à ladite au moins une source de lumière (1) et configurée pour acheminer la lumière polychromatique de ladite au moins une source de lumière (1) en direction du fluide, le corps d'extrémité (7) encapsulant l'extrémité ou les extrémités de ladite au moins une fibre optique d'éclairement (5).

7. Dispositif de mesure de teinte (10) selon la revendication précédente, dans lequel le corps d'extrémité (7) comprend un élément antireflet disposé de manière à isoler optiquement ladite au moins une fibre optique d'éclairement (5) et ladite au moins une fibre optique d'acquisition (4) entre elles, de manière à limiter la transmission directe de lumière de ladite au moins une fibre optique d'éclairement (5) à ladite au moins une fibre optique d'acquisition (4).

8. Dispositif de mesure de teinte (10) selon la revendication précédente, le corps d'extrémité (7) présentant un axe central (71), **caractérisé en ce que** les extrémités desdites au moins une fibre optique d'éclairement (5) et au moins une fibre optique d'acquisition (4) sont respectivement réparties circulairement autour de l'axe central (71) du corps d'extrémité (7) à deux distances radiales différentes de l'axe central (71) du corps d'extrémité (7).

9. Dispositif de mesure de teinte (10) selon la revendication 8, dans lequel le corps d'extrémité (7) comprenant un évidement annulaire (72) ou en forme de disque sur une face (73) configurée pour venir en vis-à-vis de la zone de mesure (140), l'évidement (72) étant aménagé dans une portion autour de l'axe central (71) et ayant un rayon compris entre lesdites deux distances radiales différentes, le corps d'extrémité (7) comprenant l'élément antireflet logé dans l'évidement (72) de sorte à isoler optiquement ladite au moins une fibre optique d'éclairement (5) et ladite au moins une fibre optique d'acquisition (4) entre elles.

10. Dispositif de mesure de teinte (10) selon l'une quelconque des revendications 1 à 9, dans lequel l'unité de calcul est en outre configurée pour déterminer le niveau de luminosité du signal lumineux reçu par le capteur de lumière (2).

11. Système de distribution de fluide (100) comprenant le circuit d'écoulement de fluide (120) et le dispositif de mesure de teinte (10) selon l'une quelconque des revendications précédentes connecté en série dans le circuit d'écoulement de fluide (120).

12. Système de distribution de fluide (100) selon la revendication précédente consistant en un système de distribution de peinture.

13. Procédé de mesure de teinte d'un fluide circulant dans un circuit d'écoulement de fluide (120) au moyen d'un dispositif de mesure de teinte (10) comprenant au moins une source de lumière (1), un capteur de lumière (2), et une unité de calcul (3), le procédé étant **caractérisé en ce qu'**il comprend les étapes suivantes :
- l'émission (E1) d'une lumière polychromatique en direction du fluide par ladite au moins une source de lumière (1) ;
- la réception (E2) par le capteur de lumière (2) du signal lumineux réfléchi sur le fluide ou transmis à travers le fluide ;
- la réalisation (E3) d'une analyse spectrale par l'unité de calcul (3) du signal lumineux reçu par le capteur de lumière (2) de sorte à déterminer une signature chromatique du fluide ;
- la comparaison (E4) de la signature chromatique du fluide à un abaque de signatures chromatiques cibles par l'unité de calcul (3) de sorte à déterminer la teinte du fluide.

14. Procédé de mesure de teinte selon la revendication précédente, comprenant la détermination d'un niveau de luminosité du signal lumineux reçu par le capteur de lumière (2).

15. Procédé de mesure de teinte selon l'une des revendications 13 ou 14, mis en œuvre lors d'une opération de changement de teinte du fluide dans le circuit d'écoulement de fluide (120), **caractérisé en ce qu'**il comprend la détermination (E5) que l'opération de changement de teinte est achevée lorsque la signature chromatique du fluide atteint une signature chromatique cible pendant une durée prédéfinie, la signature chromatique cible consistant en un sous-ensemble de l'abaque de signatures chromatiques cibles .

16. Procédé de distribution de peinture comprenant le procédé de mesure de teinte selon la revendication précédente, l'opération de changement de teinte du fluide dans le circuit d'écoulement de fluide (120) comprenant une opération de montée en teinte (MT), de changement de couleur (CC), ou de rinçage (R).
